# FASCICULE DE BREVET EUROPEEN

(11) **EP 2 720 650 B1**
(45) Date de publication et mention de la délivrance du brevet: **01.07.2015**
(21) Numéro de dépôt: 12729674.7
(22) Date de dépôt: 29.05.2012
(51) Int. Cl.: A61F 2/95, A61F 2/966

(54) **DISPOSITIF PERMETTANT DE DELIVRER UN STENT DANS UN VAISSEAU SANGUIN OU ANALOGUE**
VORRICHTUNG ZUR ABGABE EINES STENTS IN EIN BLUTGEFÄSS ODER ÄHNLICH
DEVICE FOR DELIVERING A STENT IN A BLOOD VESSEL OR SIMILAR.

(30) Priorité: 17.06.2011 FR 1101862
(43) Date de publication de la demande: 23.04.2014
(73) Titulaire: NEWCO, 75018 Paris (FR)
(72) Inventeur: PLOWIECKI, Nicolas, 95160 Montmorency (FR)
(74) Mandataire: Flavenot, Bernard
(86) Numéro de dépôt international: PCT/FR2012/000216
(87) Numéro de publication internationale: WO 2012/172191

(56) Documents cités:
- WO-A1-2009/149457
- WO-A2-2011/014814
- US-A1- 2007 088 368
- US-A1- 2009 287 292
- US-A1- 2009 318 947

## Description

La présente invention concerne les dispositifs qui permettent de délivrer, dans un vaisseau sanguin comme une artère, une endoprothèse connue dans le domaine médical sous le terme "stent", pour la mettre en place dans ce vaisseau sanguin dans le but par exemple d'obturer un anévrisme ou analogue, et plus particulièrement pour une application dans le domaine du traitement des anévrismes cérébraux ou intracrâniens.

Il est d'abord rappelé qu'un stent est un élément oblong essentiellement cylindrique, défini selon un axe longitudinal et agencé de façon à être apte à prendre deux formes, une forme repliée instable dans laquelle il a une dimension transversale d'une première valeur donnée et une forme dépliée stable dans laquelle il a une dimension transversale d'une seconde valeur donnée supérieure à la première.

Il existe plusieurs modes de réalisation possibles d'un tel stent. Mais, de façon préférentielle, il est réalisé à l'aide d'au moins un fil à mémoire de forme tissé en formant une pluralité de spires de forme sensiblement hélicoïdale se chevauchant alternativement les unes les autres pour donner un tissage.

Il existe cependant un problème, notamment avec les stents qui sont connus sous la dénomination "Flow-Diverters" (FD) quand ils sont composés de nombreux fils, par exemple de Nitinol ou d'acier élastique, tressés ensemble dans une forme cylindrique de façon que l'on puisse, par allongement, réduire leur diamètre pour les faire passer dans des micro-cathéters, puis coupés à leurs extrémités.

Les extrémités des fils coupés, formant autant de petites "aiguilles" agressives qui frottent, en la griffant, sur la surface intérieure du cathéter lors du glissement du stent à l'intérieur de ce cathéter, ce qui provoque une augmentation considérable de la friction entre le stent et la surface intérieure du cathéter et/ou qui endommage l'extrémité distale du stent.

Il est déjà connu des dispositifs qui permettent de délivrer des stents, par exemple les dispositifs décrits dans les US-A-5 449 373, US-A-5 195 984, EP-A-O 795 304, EP-A-O 832 618 et WO 96/18359, et plus particulièrement dans les US-A-2009/287292 et US-A-2009/318947.

Il existe même un dispositif permettant de délivrer un stent, qui comporte un cathéter, une pièce montée coulissante à l'intérieur de la percée traversante, un trou réalisé dans cette pièce de façon qu'il ait une ouverture d'entrée située dans la face d'extrémité de la pièce qui est tournée vers l'extrémité proximale du cathéter, ce trou étant dimensionné pour recevoir une partie de l'extrémité distale du stent quand ce dernier est dans sa forme repliée, et des moyens pour translater le stent par rapport au cathéter au moins quand ce stent est dans la percée traversante et quand la partie de son extrémité distale est enfichée dans le trou.

Cette réalisation permet d'éviter les inconvénients mentionnés ci-dessus, c'est-à-dire favorise le glissement du stent dans le cathéter en évitant l'agression des petites "aiguilles" sur la paroi intérieure du cathéter En revanche, il interdit de faire à nouveau entrer le stent dans le cathéter, quand il en est partiellement sorti et qu'il est nécessaire de mieux le positionner dans le vaisseau sanguin, notamment par rapport à l'anévrisme qui doit être traité.

En effet, avec le dispositif antérieur décrit ci-dessus, la dimension transversale de la pièce comportant le trou qui est sensiblement égale à la dimension intérieure du cathéter pour mieux y coulisser, ne permet pas, après que l'extrémité proximale du stent soit sortie du trou, de faire entrer dans le cathéter à la fois le stent et la pièce, car l'espace entre cette pièce et la paroi intérieure du cathéter n'est pas suffisant et le stent se coince alors entre le bord de l'entrée distale du cathéter et le bord de l'extrémité proximale de la pièce.

Aussi, la présente invention a-t-elle pour but de réaliser un dispositif permettant de délivrer un stent qui obvie les inconvénients mentionnés ci-dessus des dispositifs selon l'art antérieur.

Plus précisément, la présente invention a pour objet un dispositif permettant de délivrer un stent dans un vaisseau sanguin ou analogue, ayant les caractéristiques énoncées dans la revendication 1 annexée.

D'autres caractéristiques et avantages de l'invention apparaîtront au cours de la description suivante donnée en regard des dessins annexés à titre illustratif mais nullement limitatif, dans lesquels :
Les figures 1 à 3 représentent partiellement, sous forme schématique, en coupe longitudinale et partiellement en écorché, un mode de réalisation du dispositif selon l'invention permettant de délivrer un stent, respectivement dans trois configurations différentes présentées par le dispositif lors de son utilisation,
Les figures 4 et 5 représentent deux demi-vues schématiques longitudinales selon un plan diamétral d'un autre mode de réalisation du dispositif selon l'invention permettant de délivrer un stent, respectivement dans deux configurations différentes qui peuvent être prises par le dispositif lors de son utilisation,
La figure 6 représente, vu de côté, un mode de réalisation d'éléments essentiels du dispositif selon l'invention en accord avec les figures 4 et 5, et
Les figures 7 et 8 représentent un autre mode de réalisation des d'éléments essentiels du dispositif selon l'invention qui sont représentés sur la figure 6, respectivement dans deux configurations qu'ils sont aptes à prendre lors de l'utilisation du dispositif.

Il est tout d'abord précisé que, dans la présente description, si l'adverbe "sensiblement" est associé à un qualificatif d'un moyen donné, ce qualificatif doit être compris au sens strict ou approché.

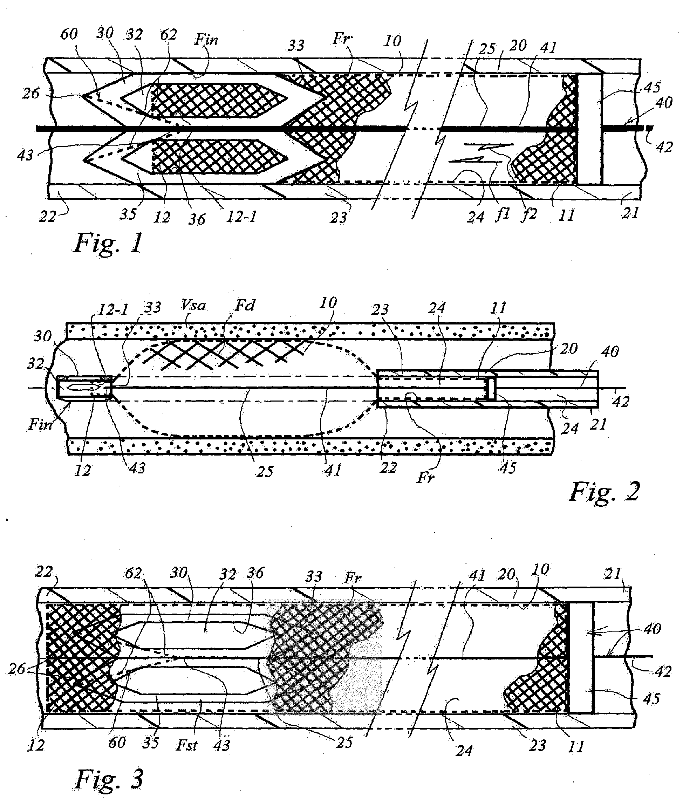

En référence aux figures annexées, la présente invention concerne un dispositif permettant de délivrer un stent 10 dans un vaisseau sanguin ou analogue Vsa, figure 2.

Le stent, connu en lui-même, est défini entre deux extrémités respectivement proximale 11 et distale 12 et est agencé de façon à être apte à prendre deux formes, une forme repliée Fr (comme sur au moins les figures 1 et 3) dans laquelle il a une dimension transversale d'une première valeur et une forme dépliée Fd (comme seulement sur la figure 2 où une partie du stent est représentée sous cette forme Fd) dans laquelle il a une dimension transversale d'une seconde valeur supérieure à la première, la longueur du stent lorsqu'il est dans sa forme dépliée Fd étant très avantageusement nettement supérieure à la seconde valeur de sa dimension transversale.

Le dispositif selon l'invention comporte un cathéter 20, connu lui aussi en lui-même, qui est défini entre une extrémité proximale 21 et une extrémité distale 22 et qui est constitué essentiellement d'un tube 23 généralement en matière plastique, comportant une percée traversante 24 de forme cylindrique, de préférence de révolution ou sensiblement de révolution, selon un axe central 25, la percée traversante étant agencée pour permettre au stent 10 d'y coulisser quand il est dans sa forme repliée Fr après y avoir été introduit, par son extrémité distale 12 par l'extrémité de la percée traversante débouchant à l'extrémité proximale 21 du cathéter 20, le stent étant maintenu dans cette forme instable, du fait qu'il est positionné dans la percée traversante 24.

Le dispositif comporte en outre une pièce 30, dont différents modes de réalisation seront donnés ci-après, montée coulissante à l'intérieur de la percée traversante 24, un trou 32 étant réalisé dans la pièce de façon qu'il ait une ouverture d'entrée 33 située dans la face d'extrémité proximale de la pièce 30, c'est-à-dire celle qui est tournée vers l'extrémité proximale 21 du cathéter 20, ce trou 32 étant agencé pour recevoir une partie 12-1 de l'extrémité distale 12 du stent 10 quand ce dernier est dans sa forme repliée Fr, figures 1, 2 et 4.

Sont aussi prévus des moyens 40 pour translater le stent 10 par rapport au cathéter 20 au moins quand ce stent est au moins en partie dans la percée traversante 24 et quand la partie 12-1 de son extrémité distale 12 est enfichée dans le trou 32 sur une longueur au plus égale à la profondeur du trou.

Il est précisé que ce trou 32 peut être un trou borgne ou préférentiellement traversant comme illustré sur les figures annexées.

Selon une caractéristique essentielle de l'invention, la pièce 30 est réalisée en un matériau à mémoire de forme, de façon qu'elle soit apte à prendre deux formes, une forme stable Fst, figures 3, 5, 6 et 8 dans laquelle elle présente une dimension transversale hors tout au plus égale à la différence entre la dimension transversale intérieure du cathéter 20 et le double de l'épaisseur de la paroi latérale du stent 10, et, une forme instable Fin, figures 1, 2, 4 et 7, dans laquelle elle présente une dimension transversale hors tout au plus égale à la dimension transversale intérieure du cathéter, optionnellement légèrement inférieure pour permettre un meilleur glissement de la pièce 30 dans le cathéter 20.

Selon une réalisation préférentielle, les moyens 40 pour translater le stent 10 par rapport au cathéter 20 comportent une âme centrale longiligne 41 d'une longueur au moins égale à celle de la percée traversante 24, définie entre une extrémité proximale 42 et une extrémité distale 43, des moyens pour commander la translation de cette âme centrale dans la percée traversante 24, une butée 45 solidaire de l'âme centrale et butant contre l'extrémité proximale du stent, et des moyens 60 pour coupler la pièce 30 avec l'âme centrale 41.

La butée 45 peut être de tout type, par exemple un simple piston pour pousser par contact le stent, ou préférentiellement un élément plus complexe comme un élément de fixation détachable lié au stent qui, en plus de la fonction de poussée par contact avec le stent, présente une fonction de fixation, d'accrochage, etc. avec l'extrémité proximale du stent 10 et pourra être détaché de façon automatique ou commandée. Cette dernière fonction est plus particulièrement utile et préférable pour réaliser un recul du stent dans le cathéter dans le sens "extrémité distale" vers "extrémité proximale" du cathéter. Ces éléments sont connus en eux-mêmes et ne seront pas plus amplement décrits ici par l'unique souci de simplifier la description.

Dans le cas où la butée 45 est conformée sensiblement en piston d'accrochage, ce dernier a une section hors tout avantageusement égale ou sensiblement égale à la section hors tout de la percée cylindrique 24.

Selon une réalisation préférentielle, les moyens 60 pour coupler la pièce 30 avec l'âme centrale 41 comportent au moins un lien souple-rigide 62, illustré schématiquement sur les figures 1 et 3, pour lier un point de cette âme centrale 41 avec une partie 26 de la pièce 30, cette partie 26 de la pièce étant située à une distance de l'ouverture d'entrée 33 du trou 32 au moins égale à la longueur de la partie 12-1 de l'extrémité distale 12 du stent 10 enfichée dans le trou 32.

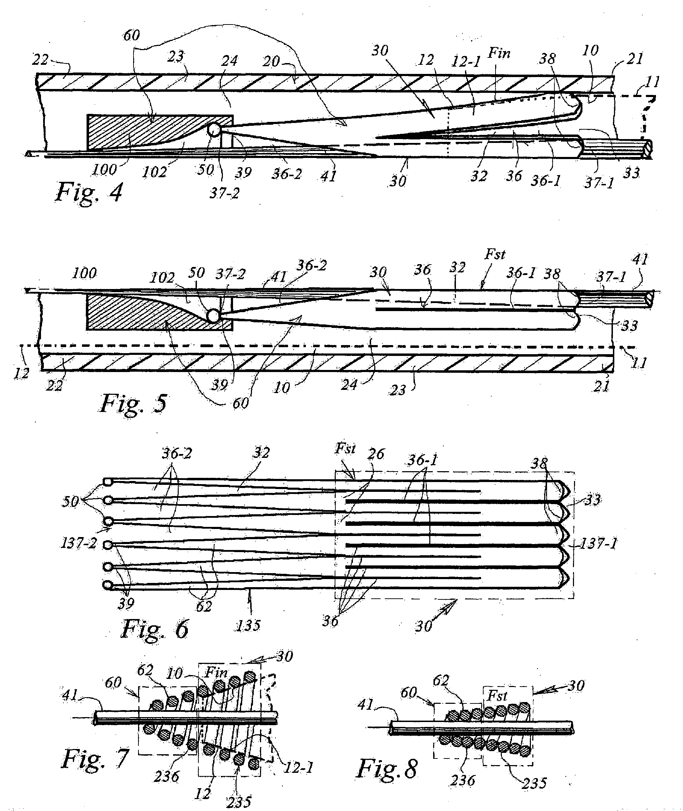

Le point de l'âme centrale 41 sur lequel est lié, par tout moyen, le lien 62 peut être situé en n'importe quel endroit de cette âme centrale, bien entendu dans des limites acceptables. Dans la réalisation illustrée sur les figures 1 à 3, ce point est situé dans le demi-espace délimité par le plan passant par l'extrémité distale de la pièce 30 et dans lequel se trouve cette pièce 30. Dans la réalisation illustrée sur les figures 4 à 6, ce point est situé dans le demi-espace délimité par le plan passant par l'extrémité distale de la pièce 30 et dans lequel ne se trouve pas cette pièce 30.

Selon une réalisation avantageuse, le trou 32 est traversant et la pièce 30 est constituée d'un manchon 35 dans la paroi duquel sont réalisées des fentes longitudinales 36.

De façon préférentielle, étant donné les dimensions d'une telle pièce, les fentes longitudinales 36 sont réalisées par découpe au laser.

Selon une réalisation possible, figures 1 à 3, ces fentes longitudinales 36 sont sensiblement en forme de losanges ou assimilables, les deux axes perpendiculaires de chaque losange étant respectivement parallèle et orthogonal à l'axe du manchon 35.

Cependant, selon une autre réalisation, figures 4 à 6, la pièce 30 et le au moins un lien 62 sont réalisés d'une seule pièce à partir d'une douille 135 qui a une longueur plus grande que celle du manchon 35 défini ci-dessus et dont l'axe longitudinal se confond avec celui du manchon.

Comme illustré sur la figure 6, la douille 135 comporte des découpes longitudinales réalisées dans sa paroi, ces découpes étant sensiblement parallèles à l'axe longitudinal de la douille et ayant des longueurs, prises selon cet axe longitudinal, inférieures à la longueur de cette douille

Comme plus particulièrement visible sur la figure 6, ces découpes longitudinales 36-1 ; 36-2 débouchent alternativement sur les faces d'extrémités respectivement proximale et distale 137-1 ; 137-2 de la douille 135. Les découpes longitudinales 36-1 qui débouchent sur la face proximale 137-1 de la douille 135 constituent en fait les fentes longitudinales 36 du manchon 35 définies ci-dessus.

Il est souligné que, comme illustré sur les figures 4 à 6, la somme des longueurs de deux découpes 36-1 et 36-2 débouchant respectivement sur les faces proximale 137-1 et distale 137-2 de la douille peut être supérieure à la longueur de la douille.

Quant aux découpes longitudinales 36-2 qui débouchent sur la face d'extrémité distale 137-2 de la douille 135, elles ont, sur une portion de leur longueur, sensiblement la forme d'un V, l'ouverture du V étant située sur la face distale 137-2 de la douille 135. Les parties de paroi de la douille qui sont comprises entre les découpes longitudinales 36-2 constituent en fait les liens 62 définis ci-avant.

En outre, les parties d'extrémité 38 de la paroi de la douille 135, comprises entre les découpes longitudinales 36-1 qui débouchent sur la face d'extrémité proximale 137-1 de cette douille sont chanfreinées, pour faciliter le glissement de la pièce 30 dans le cathéter.

Quant aux parties d'extrémité 39 de la paroi de la douille comprises entre les découpes longitudinales 36-2 qui débouchent sur la face d'extrémité distale 137-2 de cette douille 135, elles comportent optionnellement des surépaisseurs 50, comme des petites boules, utilisées pour solidariser les liens 62 avec l'âme centrale 41, par exemple comme illustré figures 4 et 5, au moyen notamment d'une bague auxiliaire 100 comportant une traversée 102 dans laquelle passe l'âme centrale 41 et dans laquelle plongent ces surépaisseurs 50. Au moyen d'un sertissage ou un collage, tous deux connus en eux-mêmes, effectué via la bague 100, les éléments "bague 100, surépaisseurs 50, âme centrale 41" sont tous solidarisés ensemble.

Il est prévu que la bague auxiliaire 100 et la douille 135 avec les découpes 36-1, 36-2 soient réalisées de préférence en un matériau comme du Nitinol ou un acier élastique, ou analogue. Le Nitinol, connu en lui-même, est un alliage à mémoire de forme constitué de Nickel et de Titane.

Cependant, selon encore une autre réalisation, figures 7 et 8, le trou 32 étant aussi traversant, la pièce 30 et le au moins un lien 62 étant aussi réalisés d'une seule pièce, cette pièce 30 est constituée par un enroulement 235 d'au moins un fil élastique 236, par exemple en Nitinol. La pluralité de fentes définies précédemment sous la référence 36 est, dans cette réalisation, remplacée par une seule fente qui n'est pas longitudinale, mais oblique par rapport à l'axe longitudinal car constituée par l'espace entre les spires, qu'elles soient jointives ou non.

Dans le mode de réalisation selon les figures 7 et 8, les parties respectivement droite et gauche de l'enroulement 235 encadrées en points interrompus constituent fonctionnellement respectivement la pièce 30 et le au moins un lien 62 définis ci-avant, les moyens pour lier l'extrémité du lien 62 avec l'âme centrale 41 étant par exemple constitués, dans ce mode de réalisation, par une fin de l'enroulement de quelques spires dont la dernière est serrée autour de l'âme centrale, en y étant en outre éventuellement collée.

Selon cette seconde réalisation, la forme instable Fin de la pièce 30, figure 7, est alors obtenue par une torsion autour de son axe longitudinal dans le sens opposé à son enroulement, provoquant ainsi son augmentation de dimension transversale par rapport à celle lorsqu'elle est dans sa forme stable Fst, figure 8.

Sur les figures 7 et 8, l'enroulement 235 est un enroulement contenu dans une enveloppe conique, mais il est bien évident que, bien que cette réalisation soit préférée, cet enroulement 235 pourrait être contenu dans une enveloppe de tout autre forme, par exemple cylindrique, notamment de révolution.

Le dispositif décrit ci-dessus pour délivrer un stent 10 dans un vaisseau sanguin Vsa comme une artère fonctionne et s'utilise de la façon suivante décrite en regard notamment de la figure 2 :

Quand un praticien veut placer un stent 10, par exemple dans une artère cérébrale d'un patient, en un endroit bien déterminé de cette artère, par exemple devant l'ouverture d'un anévrisme ou analogue, il introduit tout d'abord le cathéter 20 dans l'artère, par son extrémité distale 22, jusqu'à ce que cette extrémité distale 22 se trouve sensiblement en regard de l'endroit où doit être implanté le stent 10, ou légèrement en amont, l'extrémité proximale 21 du cathéter 20 étant, de façon connue en soi, toujours maintenue en dehors du corps du patient.

Le cathéter 20 étant mis en place comme décrit ci-dessus, l'extrémité distale 12 du sent 10 est introduite dans la percée traversante 24 du cathéter par l'extrémité proximale 21 de cette percée traversante, l'extrémité distale 12 du stent ayant été préalablement enfichée dans le trou 32 de façon que, par expansion naturelle du stent, cette extrémité distale 12 se dilate et se plaque avec une certaine force de friction contre la paroi latérale du trou 32 et oblige la pièce 30 à prendre sa position instable Fin dans laquelle elle se plaque au moins en partie contre la paroi de la percée 24, figures 1 et 4. L'introduction du stent dans la percée traversante 24 du cathéter est effectuée au moyen de l'âme centrale 41 via la pièce 30 solidaire de cette âme centrale par les liens 62.

Pour introduire complètement tout le stent 10 dans le cathéter 20, il suffit de pousser le stent 10 dans le sens de la flèche f1, figure 1, au moyen de l'âme centrale 41 via la butée 45, tout en le guidant latéralement par sécurité tant qu'il n'est pas totalement introduit dans le cathéter.

Les figures 1, 3, 4 et 5 représentent, au moins en partie, le stent 10 sous sa forme repliée Fr et entièrement contenu dans le cathéter.

La figure 2 représente le stent, en partie sous sa forme dépliée Fd dans l'artère Vsa, et en partie sous sa forme repliée dans le cathéter 20 et dans le trou 32.

L'âme centrale 41 est suffisamment poussée jusqu'à ce que la pièce 30 et une partie du stent 10 émergent de l'extrémité distale 22 du cathéter 20, comme illustré sur la figure 2, la partie restante du stent 10 demeurant maintenue dans sa forme repliée Fr dans le cathéter 20, ainsi que dans le trou 32 puisqu'il y est plaqué avec friction élastique contre sa paroi intérieure.

Lorsque le stent est suffisamment sorti de l'extrémité distale 22 du cathéter 20 en passant dans sa position dépliée Fd et en augmentant donc de diamètre, il se raccourcit et son extrémité distale sort automatiquement du trou 32 puisque la pièce 30 est maintenue fixe par rapport à l'âme centrale 41, en notant que, par précaution, le praticien a bien fait attention de garder une partie importante de l'extrémité proximale 11 du stent 10 dans le cathéter 20.

Dans cette position, toute l'extrémité distale du stent est sous la forme dépliée Fd, la pièce 30 qui n'est plus soumise à la force d'expansion de l'extrémité distale 12-1 du stent 10 reprend, par effet de mémoire de forme, sa forme stable Fst, c'est-à-dire avec une dimension transversale inférieure à celle de la percée 24 comme défini ci-avant.

Lorsque le stent est ainsi très partiellement sorti du cathéter, le praticien peut vérifier sa position par rapport à l'anévrisme, par exemple par radiographie. Au résultat de cette vérification, le praticien peut décider de rentrer le stent 10 dans le cathéter, en partie ou en totalité, afin de mieux le repositionner. Pour ce faire, il lui suffit d'agir en traction, dans le sens de la flèche f2, sur l'extrémité proximale de l'âme centrale 41 par rapport au cathéter 20.

Comme la pièce 30 a sa forme stable Fst, la paroi du stent se trouve dans l'espace libre compris entre la pièce 30 et la paroi intérieure de la percée traversante 24, figures 3 et 5, et le stent n'est nullement gêné pour rentrer dans le cathéter.

Lorsque le praticien a déterminé la position ad-hoc et idoine de l'extrémité distale du cathéter dans le vaisseau Vsa, le stent peut être délivré comme décrit ci-avant, en le poussant au moyen de l'âme centrale 41 via la butée 45, jusqu'à ce qu'il soit totalement sorti du cathéter.

Il est vrai que, dans ce denier mouvement, les fils coupés de l'extrémité distale du stent ne sont plus enfermés dans le trou 32 et peuvent entraîner les inconvénients mentionnés au préambule de la présente description. Mais, comme ces inconvénients ne se produisent que sur une très petite longueur du cathéter, ils sont insignifiants, négligeables et surtout ne gênent réellement pas le positionnement correct du stent.

Il est à noter que la pièce 30 confère au dispositif selon l'invention d'autres avantages que ceux mentionnés ci-dessus, notamment : elle facilite l'introduction du stent dans le cathéter 20, elle évite aux extrémités coupées des fils du stent d'exercer une friction par griffures sur la paroi latérale de la percée traversante 24 et elle favorise en conséquence le glissement du stent dans le cathéter.

Bien entendu, dans un souci d'efficacité et de sécurité, il sera fourni au praticien un ensemble à usage unique, sous blister stérilisé, comportant un cathéter dans lequel est déjà introduite l'âme centrale 41 sur laquelle sont fixées la butée d'accrochage 45 et la pièce 30, ainsi que le stent 10 déjà introduit dans la percée traversante 24 du cathéter 20 avec son extrémité proximale 12 logée dans le trou 32. Ceci évitera au praticien toutes les manipulations nécessaires pour obtenir l'assemblage décrit ci-dessus et minimisera tous risques supplémentaires de contamination du patient.

## Revendications

1. Dispositif permettant de délivrer un stent (10) dans un vaisseau sanguin ou analogue (Vsa), ledit stent étant défini entre deux extrémités respectivement proximale (11) et distale (12) et étant agencé de façon à être apte à prendre deux formes, une forme repliée (Fr) dans laquelle il a une dimension transversale d'une première valeur et une forme dépliée (Fd) dans laquelle il a une dimension transversale d'une seconde valeur supérieure à la première, le dispositif comportant :
• un cathéter (20) défini entre une extrémité proximale (21) et une extrémité distale (22) et constitué d'un tube (23) comportant une percée traversante (24) de forme cylindrique selon un axe central (25), ladite percée traversante étant agencée pour permettre au dit stent (10) d'y coulisser quand il est dans sa forme repliée (Fr) et après y avoir été introduit, par son extrémité distale (12), par l'extrémité de ladite percée traversante débouchant à l'extrémité proximale (21) du dit cathéter (20),
• une pièce (30) montée coulissante à l'intérieur de ladite percée traversante (24),
• un trou (32) réalisé dans ladite pièce (30) de façon qu'il ait une ouverture d'entrée (33) située dans la face d'extrémité de ladite pièce (30) qui est tournée vers l'extrémité proximale (21) du dit cathéter (20), ce dit trou (32) étant agencé pour recevoir une partie (12-1) de l'extrémité distale (12) du dit stent (10) quand il est dans sa forme repliée (Fr), et
• des moyens (40) pour translater ledit stent (10) par rapport au cathéter (20) au moins quand ce dit stent est dans ladite percée traversante (24) et quand la partie (12-1) de son extrémité distale (12) est enfichée dans ledit trou (32) sur une longueur au plus égale à la profondeur du trou, **caractérisé par le fait que** ladite pièce (30) est réalisée en un matériau à mémoire de forme, de façon qu'elle soit apte à prendre deux formes,
• (i) une forme stable (Fst) dans laquelle elle présente une dimension transversale hors tout au plus égale à la différence entre la dimension transversale intérieure du cathéter (20) et le double de l'épaisseur de la paroi latérale du stent (10), et
• (ii) une forme instable (Fin) dans laquelle elle présente une dimension transversale hors tout au moins égale à la dimension transversale intérieure du cathéter (20), optionnellement légèrement inférieure.

2. Dispositif selon la revendication 1, **caractérisé par le fait que** les moyens (40) pour translater le stent (10) par rapport au cathéter (20) comportent :
• une âme centrale longiligne (41) d'une longueur au moins égale à celle de la percée traversante (24), ladite âme centrale étant définie entre une extrémité proximale (42) et une extrémité distale (43),
• des moyens pour commander la translation de ladite âme centrale dans la percée traversante (24),
• une butée (45) solidaire de ladite âme centrale et butant contre l'extrémité proximale du stent, et
• des moyens (60) pour coupler la pièce (30) avec l'âme centrale (41).

3. Dispositif selon la revendication 2, **caractérisé par le fait que** ladite butée (45) est conformée sensiblement en un piston ayant une section hors tout sensiblement égale à la section hors tout de la percée cylindrique (24).

4. Dispositif selon l'une des revendications 2 et 3, **caractérisé par le fait que** les moyens (60) pour coupler ladite pièce (30) avec ladite âme centrale (41) comportent au moins un lien souple-rigide (62) pour lier un point de ladite âme centrale (41) avec une partie (26) de ladite pièce (30), cette partie (26) de ladite pièce étant située à une distance de l'ouverture d'entrée (33) du trou (32) au moins égale à la longueur de la partie (12-1) de l'extrémité distale (12) du stent (10) enfichée dans ledit trou (32).

5. Dispositif selon la revendication 4, **caractérisé par le fait que** ledit trou (32) est traversant et que ladite pièce (30) et le au moins un lien (62) sont réalisés d'une seule pièce, ladite pièce étant constituée d'un enroulement (235) d'au moins un fil élastique (236).

6. Dispositif selon l'une des revendications 1 à 4, **caractérisé par le fait que** ledit trou (32) est traversant et que ladite pièce (30) est constituée d'un manchon (35) dans la paroi duquel sont réalisées des fentes longitudinales (36).

7. Dispositif selon la revendication 6, **caractérisé par le fait que** lesdites fentes longitudinales (36) sont sensiblement en forme de losanges, les deux axes perpendiculaires de chaque losange étant respectivement parallèle et orthogonal à l'axe du manchon (35).

8. Dispositif selon les revendications 4 et 6, **caractérisé par le fait que** ledit lien (62) et ledit manchon (35) sont réalisés en une seule pièce dans une douille (135) ayant un axe longitudinal, ladite douille comportant des découpes longitudinales réalisées dans sa paroi sensiblement parallèles à son axe longitudinal et ayant des longueurs prises selon cet axe longitudinal inférieures à la longueur de cette douille, ces découpes longitudinales débouchant alternativement (36-1 ; 36-2) sur les faces d'extrémités respectivement proximale et distale (137-1 ; 137-2) de ladite douille, les découpes longitudinales débouchant sur la face proximale de la douille (135) constituant les fentes longitudinales (36).

9. Dispositif selon la revendication 8, **caractérisé par le fait que** les découpes longitudinales (36-2) qui débouchent sur la face d'extrémité distale (137-2) de la douille (135) ont, sur une portion de leur longueur, sensiblement la forme d'un V, l'ouverture du V étant située sur la face distale (137-2) de la douille (135).

10. Dispositif selon l'une des revendications 8 et 9, **caractérisé par le fait que** les parties d'extrémité (38) de la paroi de la douille (135) comprises entre les découpes longitudinales (36-1) qui débouchent sur la face d'extrémité proximale (137-1) de cette douille sont chanfreinées.

11. Dispositif selon la revendication 10, **caractérisé par le fait que** les moyens pour lier ledit lien souple-rigide (62) à un point de ladite âme centrale (41) comportent une bague auxiliaire (100) comportant une traversée (102) dans laquelle passe l'âme centrale (41) et dans laquelle plongent lesdites surépaisseurs (50), la bague (100), les surépaisseurs (50) et l'âme centrale (41) étant solidarisés ensemble par l'un des moyens suivants : sertissage, collage.

## Patentansprüche

1. Vorrichtung, die die Einbringung eines Stents (10) in ein Blutgefäß oder dergleichen (Vsa) ermöglicht, wobei der Stent zwischen zwei Enden, einem proximalen Ende (11) und einem distalen Ende (12), definiert ist und dafür ausgelegt ist, zwei Formen annehmen zu können, eine zusammengefaltete Form (Fr), in der er eine transversale Abmessung mit einem ersten Wert hat, und eine auseinandergefaltete Position (Fd), in der er eine transversale Abmessung mit einem zweiten Wert, der größer als der erste Wert ist, hat, wobei die Vorrichtung umfasst:
• einen Katheter (20), der zwischen einem proximalen Ende (21) und einem distalen Ende (22) definiert ist und aus einem Rohr (23) gebildet ist, das eine Durchgangsdurchlochung (24) in zylindrischer Form längs einer Mittelachse (25) aufweist, wobei die Durchgangsdurchlochung dafür ausgelegt ist, dem Stent (10) zu ermöglichen, durch sie zu gleiten, wenn er in der zusammengefalteten Form (Fr) ist und nachdem er in sie mit seinem distalen Ende durch das Ende der Durchgangsdurchlochung, die in das proximale Ende (21) des Katheters (20) mündet, eingeführt worden ist,
• ein Element (30), das in der Durchgangsdurchlochung (24) gleitend montiert ist,
• ein Loch (32), das in dem Element (30) in der Weise verwirklicht ist, dass es eine Einlassöffnung (33) besitzt, die sich in der Stirnfläche des Elements (30) befindet, die dem proximalen Ende (21) des Katheters (20) zugewandt ist, wobei dieses Loch (32) dafür ausgelegt ist, einen Teil (12-1) des distalen Endes (12) des Stents (10) aufzunehmen, wenn er in seiner zusammengefalteten Form (Fr) ist, und
• Mittel (40) zum translatorischen Bewegen des Stents (10) in Bezug auf den Katheter (20) wenigstens dann, wenn sich dieser Stent in der Durchgangsdurchlochung (24) befindet und wenn der Teil (12-1) seines distalen Endes (12) in das Loch (32) über eine Länge gesteckt ist, die höchstens gleich der Tiefe des Lochs ist,
**dadurch gekennzeichnet, dass** das Element (30) aus einem Formerinnerungsmaterial verwirklicht ist, derart, dass es zwei Formen annehmen kann,
• (i) eine stabile Form (Fst), in der es eine transversale Abmessung über alles aufweist, die höchstens gleich dem Unterschied zwischen der inneren transversalen Abmessung des Katheters (20) und der doppelten Dicke der Seitenwand des Stents (10) ist, und
• (ii) eine instabile Form (Fin), in der es eine transversale Abmessung über alles aufweist, die wenigstens gleich der inneren transversalen Abmessung des Katheters (20), optional etwas kleiner, ist.

2. Vorrichtung nach Anspruch 1, **dadurch gekennzeichnet, dass** die Mittel (40) zum translatorischen Bewegen des Stents (10) in Bezug auf den Katheter (20) umfassen:
• eine langgestreckte Mittelseele (41) mit einer Länge, die wenigstens gleich jener der Durchgangsdurchlochung (24) ist, wobei die Mittelseele zwischen einem proximalen Ende (42) und einem distalen Ende (43) definiert ist,
• Mittel zum Steuern der translatorischen Bewegung der Mittelseele in der Durchgangsdurchlochung (24),
• einen Anschlag (45), der mit der Mittelseele fest verbunden ist und an dem proximalen Ende des Stents anliegt, und
• Mittel (60) zum Koppeln des Elements (30) mit der Mittelseele (41).

3. Vorrichtung nach Anspruch 2, **dadurch gekennzeichnet, dass** der Anschlag (45) im Wesentlichen wie ein Kolben geformt ist, der einen Querschnitt über alles besitzt, der im Wesentlichen gleich dem Querschnitt über alles der zylindrischen Durchlochung (24) ist.

4. Vorrichtung nach einem der Ansprüche 2 und 3, **dadurch gekennzeichnet, dass** die Mittel (60) zum Koppeln des Elements (30) mit der Mittelseele (41) wenigstens eine elastisch-starre Verbindung (62) umfassen, um einen Punkt der Mittelseele (41) mit einem Teil (26) des Elements (30) zu verbinden, wobei sich dieser Teil (26) des Elements in einem Abstand von der Einlassöffnung (33) des Lochs (32) befindet, der wenigstens gleich der Länge des in das Loch (32) gesteckten Teils (12-1) des distalen Endes (12) des Stents (10) ist.

5. Vorrichtung nach Anspruch 4, **dadurch gekennzeichnet, dass** das Loch (32) ein Durchgangsloch ist und dass das Element (30) und die wenigstens eine Verbindung (62) einteilig verwirklicht sind, wobei das Element aus einer Wicklung (235) wenigstens eines elastischen Fadens (236) gebildet ist.

6. Vorrichtung nach einem der Ansprüche 1 bis 4, **dadurch gekennzeichnet, dass** das Loch (32) ein Durchgangsloch ist und dass das Element (30) aus einer Muffe (35) gebildet ist, in deren Wand Längsschlitze (36) verwirklicht sind.

7. Vorrichtung nach Anspruch 6, **dadurch gekennzeichnet, dass** die Längsschlitze (36) im Wesentlichen die Form von Rauten haben, wobei die beiden senkrechten Achsen jeder Raute zu der Achse der Muffe (35) parallel bzw. senkrecht sind.

8. Vorrichtung nach den Ansprüchen 4 und 6, **dadurch gekennzeichnet, dass** die Verbindung (62) und die Muffe (35) in einer Hülse (135), die eine Längsachse besitzt, einteilig verwirklicht sind, wobei die Hülse longitudinale Ausschnitte aufweist, die in ihrer Wand im Wesentlichen parallel zu ihrer Längsachse verwirklicht sind und Längen längs dieser Längsachse besitzen, die kleiner sind als die Länge dieser Hülse, wobei diese longitudinalen Ausschnitte (36-1; 36-2) abwechselnd in die proximale bzw. die distale Stirnfläche (137-1; 137-2) der Hülse münden, wobei die longitudinalen Ausschnitte in die proximale Fläche der Hülse (135) münden und die Längsschlitze (36) bilden.

9. Vorrichtung nach Anspruch 8, **dadurch gekennzeichnet, dass** die longitudinalen Ausschnitte (36-2), die in die distale Stirnfläche (137-2) der Hülse (135) münden, auf einem Teil ihrer Länge im Wesentlichen die Form eines V haben, wobei sich die Öffnung des V in der distalen Fläche (137-2) der Hülse (135) befindet.

10. Vorrichtung nach einem der Ansprüche 8 und 9, **dadurch gekennzeichnet, dass** die Endteile (38) der Wand der Hülse (135), die zwischen den longitudinalen Ausschnitten (36-1) enthalten sind, die in die proximale Stirnfläche (137-1) dieser Hülse münden, angefast sind.

11. Vorrichtung nach Anspruch 10, **dadurch gekennzeichnet, dass** die Mittel zum Verbinden der elastisch-starren Verbindung (62) mit einem Punkt der Mittelseele (41) einen Hilfsring (100) umfassen, der einen Durchgang (102) aufweist, durch den die Mittelseele (41) verläuft und in den die Überdicken (50) ragen, wobei der Ring (100), die Überdicken (50) und die Mittelseele (41) durch eines der folgenden Mittel fest miteinander verbunden sind: Falzen, Kleben.

## Claims

1. A device enabling a stent (10) to be delivered into a blood vessel or the like (Vsa), said stent being defined between two ends, respectively a proximal end (11) and a distal end (12), and being arranged in such a manner as to be suitable for taking up two shapes, a folded shape (Fr) in which it has a transverse dimension of a first value, and a deployed shape (Fd) in which it has a transverse dimension of a second value greater than the first, the device comprising:
a catheter (20) defined between a proximal end (21) and a distal end (22) and constituted by a tube (23) having a through bore (24) of cylindrical shape along a central axis (25), said through bore being arranged so as to enable said stent (10) to slide therealong when it is in its folded state (Fr), and after being inserted therein via its distal end (12) through the end of said through bore that opens out into the proximal end (21) of said catheter (20);
• a part (30) slidably mounted inside said through bore (24);
• a hole (32) made in said part (30) so as to have an inlet opening (33) situated in the end face of said part (30) that faces towards the proximal end (21) of said catheter (20), said hole (32) being arranged to receive a portion (12-1) of the distal end (12) of said stent (10) when it is in its folded shape (Fr); and
• mean (40) for moving said stent (10) in translation relative to the catheter (20) at least when said stent is in said through bore (24) and when the portion (12-1) of its distal end (12) is plugged into said hole (32) over a length not less than the depth of the hole;
the device being **characterized by** the fact that said part (30) is made of a shape memory material in such a manner as to be suitable for taking up two shapes:
i) a stable shape (Fst) in which it presents an overall transverse dimension no greater than the difference between the inside transverse dimension of the catheter (20) and twice the thickness of the side wall of the stent (10); and
ii) an unstable shape (Fin) in which it presents an overall transverse dimension not less than the inside transverse dimension of the catheter (20), and possibly slightly smaller.

2. A device according to claim 1, **characterized by** the fact that the means (40) for moving the stent (10) in translation relative to the catheter (20) comprise:
• an elongate central core (41) of length not less than the length of the through bore (24), said central core being defined between a proximal end (42) and a distal end (43);
• means for controlling movement of said central core in translation in the through bore (24);
• an abutment (45) secured to said central core and abutting against the proximal end of the stent; and
• means (60) for coupling the part (30) with the central core (41).

3. A device according to claim 2, **characterized by** the fact that said abutment (45) is substantially shaped as a piston having an overall section that is substantially equal to the overall section of the cylindrical bore (24).

4. A device according to claim 2 or claim 3, **characterized by** the fact that the means (60) for coupling said part (30) with said central core (41) comprise at least one flexible-rigid tie (62) for connecting a point of said central core (41) with a portion (26) of said part (30), said portion (26) of said part being at a distance from the inlet opening (33) of the hole (32) that is not less than the length of the portion (12-1) of the distal end (12) of the stent (10) plugged into said hole (32).

5. A device according to claim 4, **characterized by** the fact that said hole (32) is a through hole and that said part (30) and the at least one tie (62) are made as a single part, said part being constituted by a winding (235) of at least one spring wire (236).

6. A device according to any one of claims 1 to 4, **characterized by** the fact that said hole (32) is a through hole and said part (30) is constituted by a sleeve (35) having longitudinal slots (36) made in its wall.

7. A device according to claim 6, **characterized by** the fact that said longitudinal slots (36) are substantially lozenge-shaped, the two perpendicular axes of each lozenge shape being respectively parallel and orthogonal to the axis of the sleeve (35).

8. A device according to claims 4 and 6, **characterized by** the fact that said tie (62) and said sleeve (35) are made as a single part in a bushing (135) having a longitudinal axis, said bushing having longitudinal cutouts made in its wall substantially parallel to its longitudinal axis and having lengths along said longitudinal axis that are shorter than the length of the bushing, said longitudinal cutouts opening out in alternation (36-1; 36-2) into the proximal and distal end faces (137-1; 137-2) respectively of said bushing, the longitudinal cutouts opening out into the proximal face of the bushing (135) constituting the longitudinal slots (36).

9. A device according to claim 8, **characterized by** the fact that the longitudinal cutouts (36-2) that open out into the distal end face (137-2) of the bushing (135) are substantially V-shaped over a portion of their length, the openings of the V-shapes being situated in the distal face (137-2) of the bushing (135).

10. A device according to claim 8 or claim 9, **characterized by** the fact that the end portions (38) of the wall of the bushing (135) that lie between the longitudinal cutouts (36-1) opening out into the proximal end face (137-1) of the bushing are chamfered.

11. A device according to claim 10, **characterized by** the fact that the means for connecting said flexible-rigid tie (62) to a point of said central core (41) comprise an auxiliary ring (100) having a passage (102) through which the central core (41) passes and into which said extra thicknesses (50) penetrate, the ring (100), the extra thicknesses (50), and the central core (41) being secured together by one of the following means: crimping, adhesive.
